# EUROPEAN PATENT APPLICATION

(11) **EP 4 438 728 A1**
(43) Date of publication of application: **02.10.2024**
(21) Application number: 22895722.1
(22) Date of filing: 21.11.2022
(51) Int. Cl.: C12N 15/10, C12Q 1/02, C40B 40/06

(54) **METHOD FOR CONSTRUCTING MUTANT LIBRARY POOL**

(30) Priority: 22.11.2021 JP 2021189096
(71) Applicant: Kao Corporation, Tokyo 103-8210 (JP)
(72) Inventor: HIOKI, Takahiro, Wakayama-shi, Wakayama 640-8580 (JP); TERAI, Mika, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2022/042989
(87) International publication number: WO 2023/090441

(57) **Abstract**

To provide a method for preparing a highly diverse mutation library pool at a low price easily. A method for preparing a mutation library pool, comprising the following steps (a) to (c): (a) subjecting a template DNA and each of a plurality of mutagenic primer pairs to DNA synthesis reaction with a DNA polymerase to acquire DNA fragments having 5'-protruding ends, wherein each of the mutagenic primer pairs anneals to the template DNA, primers of each primer pair have regions complementary to each other, and at least one of the primers comprises a mutagenic site; (b) allowing a DNA ligase to act on the DNA fragments obtained in step (a); and (c) transforming host cells with the DNAs obtained in step (b).

## Description

### Field of the Invention

The present invention relates to a method for preparing a mutant library pool.

### Background of the Invention

DNA mutation libraries in which diverse mutations are introduced into template DNAs are used to analyze and improve functions which DNA sequences such as proteinencoding sequences and promoter sequences have. Mutation libraries include methods using individual mutation DNAs independently and methods using mutation library pools which are mixtures of diverse mutated DNAs. Since the automation of experiments and the development of highthroughput assay systems have progressed in recent years, sequences can be screened for structural analysis or functional improvement using highly diverse mutation library pools. A method for preparing a highly diverse mutation library pool widely containing mutations of interest at a low price easily has therefore been desired.

As a method for preparing a large-scale mutation library pool, error-prone PCR (Non Patent Literature 1) is the most widely used. High-performance reagents for error-prone PCR are sold by companies, and mutation library pools can be easily prepared at a low price. A problem is, however, that error-prone PCR very rarely introduces mutations into adjacent bases at the same time, and cannot access very many mutated sequences (Non Patent Literature 2). When this problem is solved, massively parallel oligonucleotide synthesis (Non Patent Literature 2) has been considered to be effective means in recent years. Massively parallel oligonucleotide synthesis enables synthesizing mutation library pools containing mutations of interest widely. In the preparation of a large-scale mutation library pool, however, massively parallel oligonucleotide synthesis remains more expensive than error-prone PCR, and it is particularly expensive and difficult to synthesize mutation library pools on a scale of more than 10⁶. For example, a PCR technique using a single-stranded circular DNA as a template (Non Patent Literature 3) and a technique using Golden Gate Cloning (for example, Non Patent Literature 4) have meanwhile been reported as techniques for preparing mutation library pools widely containing mutations of interest at a low price. The techniques however still have room for improvement in ease from the viewpoints that the sequence of the template is limited in the technique by the PCR using a single-stranded circular DNA as the template, and the design of the oligo DNAs to be used is complicated in the technique using Golden Gate Cloning. In these techniques, the length of an applicable sequence to be modified is not clearly limited, and the mutagenesis into a large region may reduce the efficiency.

As a method for single mutagenesis, an easy site-directed mutagenesis method by PCR using a complementary primer pair is meanwhile known (Non Patent Literatures 5 and 6 and Patent Literature 1), but a method for preparing a large-scale mutation library pool by PCR using a complementary primer pair remains unknown.

### (Patent Literature 1) JP-B-5390076

(Non Patent Literature 1) Cirino, Patrick C., Kimberly M. Mayer, and Daisuke Umeno. Directed Evolution Library Creation. Humana Press, 2003.3-9.
(Non Patent Literature 2) Oeling, David, et al. ACS Synthetic Biology 7.9 (2018): 2317-2321.
(Non Patent Literature 3) Wrenbeck, Emily E., et al. Nature Methods 13.11 (2016): 928-930.
(Non Patent Literature 4) Nedrud, David, Willow Coyote-Maestas, and Daniel Schmidt. Proteins: Structure, Function, and Bioinformatics (2021).
(Non Patent Literature 5) Braman, Jeffrey, Carol Papworth, and Alan Greener. In Vitro Mutagenesis Protocols (1996): 31-44.
(Non Patent Literature 6) Zheng, Lei, Ulrich Baumann, and Jean-Louis Reymond. Nucleic Acids Research 32.14 (2004): e115-e115.

### Summary of the Invention

In one embodiment, the present invention provides a method for preparing a mutation library pool, comprising the following steps (a) to (c):
(a) subjecting a template DNA and each of a plurality of mutagenic primer pairs to DNA synthesis reaction with a DNA polymerase to acquire DNA fragments having 5'-protruding ends, wherein each of the mutagenic primer pairs anneals to the template DNA, primers of each primer pair have regions complementary to each other, and at least one of the primers comprises a mutagenic site;
(b) allowing a DNA ligase to act on the DNA fragments obtained in step (a); and
(c) transforming host cells with the DNAs obtained in step (b).

In another embodiment, the present invention provides a method for preparing a mutation library pool, comprising the following steps (a') to (c):
(a') subjecting a template DNA and a mutagenic primer pair to DNA synthesis reaction with a DNA polymerase to acquire DNA fragments having 5'-protruding ends, wherein the template DNA is a mixture of a plurality of DNAs having partially different nucleotide sequences, the mutagenic primer pair anneals to the template DNA, primers of each primer pair have regions complementary to each other, and at least one of the primers comprises a mutagenic site;
(b) allowing a DNA ligase to act on the DNA fragments obtained in step (a'); and
(c) transforming host cells with the DNAs obtained in step (b).

In another embodiment, the present invention provides mutation library pools prepared by the above-mentioned methods for preparing mutation library pools.

In another embodiment, the present invention provides a method for screening for DNA having a desired property, comprising a step of selecting DNA having the desired property from the mutation library pool prepared by the above-mentioned methods for preparing a mutation library pool using the desired property as an indicator.

### Brief Description of Drawings

[FIG. 1] FIG. 1 is a schematic diagram of the design of a primer pair for saturation mutagenesis of the histidine residue at position 24 of β-lactamase.
[FIG. 2] FIG. 2 is a figure showing the appearance frequency of the DNA triplets at each amino acid residue position of YR288dRT (α-amylase mutant) in a single mutation library pool of YR288dRT.
[FIG. 3] FIG. 3 is a figure showing the appearance frequency of the DNA triplets at each amino acid residue position of YR288dRT in a double mutation library pool of YR288dRT.
[FIG. 4] FIG. 4 is a mapping diagram of mutations which can be introduced by single base substitution at each amino acid residue position of YR288dRT by error-prone PCR.

### Detailed Description of the Invention

The "mutation library pool" as used herein refers to a mixture of DNAs containing different nucleotide sequences due to different mutations or a mixture of bacterial cells or fungal cells having the DNAs. The mutation library pool prepared by the method of the present invention is preferably a mixture of plasmid DNAs containing different nucleotide sequences due to different mutations or a mixture of bacterial cells or fungal cells having the plasmid DNAs. For example, a "mutation library pool" of a protein can be a mixture of plasmid DNAs containing DNAs encoding the protein and consisting of different nucleotide sequences due to different mutations or a mixture of bacterial cells or fungal cells having the plasmid DNAs. A "single mutation library pool" of a protein means a mutation library pool including its individual constituent members having a mutation corresponding to the substitution of a single amino acid residue in DNA encoding the protein among the above-mentioned mutation library pool. A "double mutation library pool" of a protein means the above-mentioned mutation library pool including it individual constituent members having mutations corresponding to the substitution of any two amino acid residues in the DNA encoding the protein among the above-mentioned mutation library pool.

The "saturation mutagenesis" as used herein refers to the introduction of various mutations which can occur in the nucleotide sequence at a specified position of DNA. If the DNA encodes a protein, various mutations which can occur to an amino acid residue at a specified position of the protein are consequently introduced.

The abbreviations for bases and amino acid residues as used herein are in accordance with the IUPAC notation, approved officially. The "deoxyribonucleic acid (DNA)" as used herein includes not only double-stranded DNA but also single-stranded DNAs constituting the double-stranded DNA, namely the sense strand and the antisense strand.

The "complementary" to a nucleotide sequence as used herein does not only mean that one is completely complementary to the nucleotide sequence, but also means that one may have preferably 80% or more, more preferably 90% or more, further more preferably 95% or more, still more preferably 98% or more, and further still preferably 99% or more nucleotide sequence identity with the sequence complementary to the nucleotide sequence. The identity between nucleotide sequences can be determined by algorithm such as BLAST.

Various operations such as enzyme treatment for synthesizing, cleaving, deleting, adding, or ligating DNA; the isolation, the purification, and the selection of DNA; and the preparation of recombinant DNA which can be adopted here can all be performed according to ordinary methods (for example, refer to *"*Bunshi-idengaku Jikken-hou (Method for Molecular Genetic Experiment)", published by Kyoritsu Shuppan Co., Ltd. in 1993; " PCR Technology", published by TAKARA SHUZO CO., LTD. in 1990; Science, 224, 1431 (1984); Biochem. Biophys. Res. Comm., 130, 692 (1985); Proc. Natl. Acad. Sci., USA., 80,5990 (1983); Moplecular Cloning, by T. Maniatis et al., Cold Spring Harbor Laboratory (1982); and the like).

The contents of all of Patent Literatures, Non Patent Literature, and other publications cited herein are incorporated herein in their entirety as reference.

The present invention provides a method for preparing a highly diverse mutation library pool widely containing mutations of interest at a low price easily.

The present inventors extensively examined a method for preparing a mutation library pool and consequently found that a template DNA and each of a plurality of complementary primer pairs for introducing different mutations are subjected to PCR, the obtained DNA fragments are mixed, and host cells can be transformed with the mixture to prepare a mutation library pool. Meanwhile, it was proved that a problem is that the efficiency in transformation with the DNA fragments amplified by PCR using the complementary primer pairs is insufficient for constructing a mutation library pool requiring diversity. The present inventors further repeated examination to solve the problem and consequently found that subjecting DNA fragments amplified by PCR using complementary primer pairs to ligation reaction enables the transformation efficiency to be greatly improved. Furthermore, it was found that a mutation library pool dramatically improved in diversity can be prepared by the use of a mutation library pool prepared beforehand as template DNA.

According to the method of the present invention, a highly diverse and comprehensive or semicomprehensive mutation library pool widely containing mutations interest can be prepared. The method of the present invention basically incudes easy techniques, namely DNA synthesis reaction using complementary primer pairs, ligation reaction, and transformation, and enables a mutation library pool to be prepared at a low price.

The present invention provides a method for preparing a mutation library pool. The method includes the following steps (a) to (c):
(a) subjecting a template DNA and each of a plurality of mutagenic primer pairs to DNA synthesis reaction with a DNA polymerase to acquire DNA fragments having 5'-protruding ends, wherein each of the mutagenic primer pairs anneals to the template DNA, primers of each primer pair have regions complementary to each other, and at least one of the primers comprises a mutagenic site;
(b) allowing a DNA ligase to act on the DNA fragments obtained in step (a); and
(c) transforming host cells with the DNAs obtained in step (b).

Step (a) of the present invention is a step of subjecting a template DNA and each of a plurality of mutagenic primer pairs to DNA synthesis reaction with a DNA polymerase to acquire DNA fragments having 5'-protruding ends. The DNA fragments mutated at specified sites are obtained by such a step.

The "template DNA" used in the method of the present invention may contain a DNA of interest to be mutated. The type and the derivation of the DNA of interest are not limited. Examples of the DNA of interest include DNA encoding a promoter and DNA encoding a protein, and preferable examples include DNA encoding a protein. The DNA of interest may have any length, and for example, the length thereof is preferably 15 bases or more, more preferably 60 bases or more, further more preferably 150 bases or more, still more preferably 300 bases or more, further still more preferably 450 bases or more, further still more preferably 600 bases or more, and further still more preferably 900 bases or more, and the length is equal to or shorter than the full length of the DNA of interest. On the amino acid sequence length basis, this is preferably 5 amino acid residues or more, more preferably 20 amino acid residues or more, further more preferably 50 amino acid residues or more, still more preferably 100 amino acid residues or more, further still preferably 150 amino acid residues or more, further still preferably 200 amino acid residues or more, and further still preferably 300 amino acid residues or more, and this is equal to or shorter than the full length of the amino acid sequence encoded by the DNA of interest. The DNA of interest can be acquired, for example, from genomic DNA, mitochondrial genomic DNA, chloroplast genomic DNA, plasmid DNA, viral genomic DNA, or synthetic DNA containing the DNA of interest by an ordinary genetic engineering technique.

The template DNA may be circular DNA, and is preferably double-stranded circular DNA. As long as the double-stranded circular DNA can have circular structure, the double-stranded circular DNA also includes DNA in which one or both of the strands are nicked or gapped and partially single-stranded DNA. Specific examples of the circular DNA include plasmid DNA and cosmid DNA, and plasmid DNA is preferably used in the method of the present invention. Preferable examples of the plasmid DNA include, but not limited to, pET22b(+), pBR322, pBR325, pUC57, pUC118, pUC119, pUC18, pUC19, pBluescript, pHA3040SP64, pHSP64R or pASP64 (JP-B-3492935), pHY300PLK (Jpn J Genet, 1985, 60: 235-243), pAC3 (Nucleic Acids Res, 1988, 16:8732), pUB110 (J Bacteriol, 1978, 134: 318-329), and pTA10607 (Plasmid, 1987, 18:8-15). A marker gene for selecting transformants in step (c), described below, (for example, a resistance gene to a drug such as ampicillin, neomycin, kanamycin, or chloramphenicol) may be incorporated into the plasmid. Alternatively, if an auxotroph is used as the host, a gene encoding an enzyme for synthesizing required nutrition may be incorporated as the marker gene. Alternatively, if selection medium requiring specified metabolism for growth, a gene related to the metabolism may be incorporated as the marker gene.

As the template DNA, one template DNA may be used, or a mixture of a plurality of DNAs having partially different nucleotide sequences, specifically partially different nucleotide sequences from the nucleotide sequence of the DNA of interest, may be used. The number of the plurality of DNAs used here is at least 2, preferably 50 or more, more preferably 100 or more, further more preferably 300 or more, still more preferably 500 or more, further still more preferably 1,000 or more, further still more preferably 2,000 or more, further still more preferably 3,000 or more, further still more preferably 5,000 or more, and further still more preferably 10,000 or more, and the number is preferably equal to or less than the number of all the mutants which can be theoretically generated from the nucleotide sequence of the DNA of interest. As the number of the template DNAs increases, the obtained mutation library pool becomes more highly diverse. The partially different sequence means that at least one base in the sequence is different.

Alternatively, a mutation library pool prepared by the method of the present invention or a mutation library pool prepared by a known method (for example, error-prone PCR) can be also used as the template DNA, which enables a more highly diverse mutation library pool to be prepared.

Each of the "mutagenic primer pairs" used in the method of the present invention anneals to the template DNA, the primers of each primer pair have regions complementary to each other, and at least one of the primers contains a mutagenic site. The phrase "having regions complementary to each other" as used herein means that a first primer and a second primer constituting the primer pair have partially or entirely complementary sequences to each other. The primer pair having regions complementary to each other used herein may be referred to as a complementary primer pair. The regions complementary to each other of the primer pair are preferably complementary also to the template DNA. Preferably only parts of the sequences of the first primer and the second primer are complementary to each other, more preferably at least parts of the sequences on the 5'-end sides of each primer are complementary to each other, and further preferably regions including the 5'-end of each primers are complementary to each other from the viewpoint of reducing primer dimers. The length of the complementary sequences is, but not limited to, preferably from 9 to 30 bases, more preferably from 12 to 24 bases, and further preferably from 15 to 18 bases.

The "mutagenic site" is a site for introducing a mutation into the template DNA, specifically a DNA of interest in the template DNA, and consists of the nucleotide sequence corresponding to the mutation to be introduced. The mutation may be any of substitution, insertion, and deletion, and may be any one thereof or a combination of any two or more thereof. If the mutation to be introduced is substitution, the nucleotide sequence corresponding to the mutation may be a nucleotide sequence to be substituted in the DNA of interest or a nucleotide sequence complementary thereto. Alternatively, if the mutation to be introduced is insertion, the nucleotide sequence corresponding to the mutation may be a nucleotide sequence to be inserted into the DNA of interest or a nucleotide sequence complementary thereto. When the substitution or the insertion is introduced, a plurality of mutations can also be introduced at once using at least one mixed base selected from the group consisting of R, M, W, S, Y, K, H, B, D, V, and N as a base constituting the nucleotide sequence corresponding to the mutation. Alternatively, when the mutation to be introduced is deletion, the nucleotide sequence corresponding to the mutation may be a nucleotide sequence obtained by connecting a nucleotide sequence adjacent to the 5'-end of the nucleotide sequence to be deleted from the DNA of interest and a nucleotide sequence adjacent to the 3'-end of the deleted nucleotide sequence or the nucleotide sequence complementary thereto may be adopted as the nucleotide sequence corresponding to the mutation. When the mutation to be introduced is substitution or insertion, the length of the mutagenic site is not particularly limited, and preferably from 1 to 21 bases, more preferably from 1 to 18 bases, further more preferably from 3 to 9 bases, when the mutation to be introduced is deletion, the mutagenic site may have any length.

The mutagenic site may be contained in at least one of the first primer and the second primer constituting the mutagenic primer pair, or may be contained in both. If the mutagenic site is contained in only one primer, the mutagenic site is contained in a region where the one primer is not complementary to the other primer, and is preferably contained on the 3' side of a region where the one primer is complementary to the other primer. Meanwhile, if the mutagenic site is contained in both primers, the mutagenic site is contained in the complementary region of both primers, and consists of sequences complementary to each other. The mutagenic site is preferably contained in any one primer of the mutagenic primer pair from the viewpoint of cost. The primer containing the mutagenic site preferably includes a nucleotide sequence for annealing to the template DNA on the 3'-end side(s) of the mutagenic site. The length of the nucleotide sequence is, not particularly limited to, and preferably from 5 to 30 bases, more preferably from 10 to 25 bases, and further more preferably from 15 to 20 bases.

Preferable examples of the mutagenic primer pair include, but not limited to, a primer pair which anneals to the template DNA, of which primers have complementary nucleotide sequences complementary to each other with around 15 bases at the 5'-end, wherein one primer contains a mutagenic site on the 3'-end side of the region complementary to the other primer, and further contains a nucleotide sequence with around 17 bases on the 3'-end side thereof, and the other primer contains a nucleotide sequence with around 9 bases on the 3'-end side of the region complementary to the one primer.

In the method of the present invention, a plurality of mutagenic primer pairs are used. The number of the plurality of primer pairs used here is at least 2, preferably 10 or more, more preferably 20 or more, further more preferably 30 or more, still more preferably 50 or more, further still more preferably 70 or more, further still more preferably 100 or more, further still more preferably 150 or more, further still more preferably 200 or more, and further still more preferably 300 or more, and the number is preferably equal to or less than the number of types of mutations which can be theoretically introduced into the nucleotide sequence of the DNA of interest. The plurality of mutagenic primer pairs are for introducing different mutations into the DNA of interest, and are preferably for introducing mutations at different positions of the DNA of interest. The number of the plurality of mutagenic primer pairs is therefore at least 2, preferably 10 or more, more preferably 20 or more, further more preferably 30 or more, still more preferably 50 or more, further still more preferably 70 or more, further still more preferably 100 or more, further still more preferably 150 or more, further still more preferably 200 or more, and further still more preferably 300 or more for the DNA of interest, and the number is preferably equal to or less than the number of types of mutations which can be theoretically introduced into the nucleotide sequence of the DNA of interest. The number of points at which the plurality of mutagenic primer pairs introduce mutations into the DNA of interest is at least 2, preferably 10 or more, more preferably 20 or more, further more preferably 30 or more, still more preferably 50 or more, further still more preferably 70 or more, further still more preferably 100 or more, further still more preferably 150 or more, further still more preferably 200 or more, and further still more preferably 300 or more, and the number is equal to or less than the number of points at which the mutations can be theoretically introduced into the nucleotide sequence of the DNA of interest.

The mutagenic primer pairs can be appropriately designed based on the nucleotide sequence of the DNA of interest and the nucleotide sequences corresponding to the mutations to be introduced. The primers may have lengths which enable specific annealing and strand elongation, and examples of the primers include primers having strand lengths of preferably from 10 to 100 bases, more preferably from 15 to 50 bases, and further more preferably from 20 to 40 bases. As long as the primers enable specific annealing and strand elongation, the primers may contain mismatches with the template DNA. The primers can be DNAs or RNAs, and may be synthesized or natural.

The "DNA synthesis reaction with a DNA polymerase" in the method of the present invention can be PCR amplification reaction with various DNA polymerases or DNA replication reaction with various DNA polymerases. The reaction can be performed by any method well-known to those skilled in the art. As long as the DNA polymerase which can be used in the method of the present invention can synthesize a DNA strand having a nucleotide sequence complementary to DNA using the DNA as a template, the DNA polymerase may be any DNA polymerase, and is preferably DNA polymerase having proofreading activity and high fidelity from the viewpoint of reducing the mutagenesis which is not interested. Such DNA polymerase is marketed, and for example, KOD DNA polymerase (TOYOBO Co., Ltd.), PrimeSTAR(Registered Trademark) DNA polymerase (Takara Bio Inc.), PfuUltra(Registered Trademark) DNA polymerase (Agilent Technologies Japan, Ltd.), and Platinum(Registered Trademark) SuperFi II DNA polymerase (Thermo Fisher Scientific K.K.) can be used.

The DNA synthesis reaction with a DNA polymerase can be preferably performed by PCR. The PCR can be performed with commercially available reagents and equipments for PCR according to an ordinary method. The conditions for PCR is not particularly limiter, and optimal conditions may be set for each PCR. Examples thereof include the following conditions.
1) Heat denaturation of double-stranded DNA into single-stranded DNAs: Heating is performed usually at from around 93 to 95°C usually for from around 3 seconds to 1 minute.
2) Annealing: Heating is performed usually at from around 50 to 60°C usually for from around 10 seconds to 1 minute.
3) DNA elongation reaction: Heating is performed usually at from around 60 to 74°C usually for from around 10 seconds to 5 minutes.

The above-mentioned reactions from 1) to 3) are defined as one cycle, usually 30 or more cycles and preferably 35 or more cycles and usually 50 or less cycles and preferably 45 or less cycles may be performed.

The DNA synthesis reaction with a DNA polymerase is performed with each of the plurality of mutagenic primer pairs. DNA fragments having different mutations introduced are consequently obtained for each DNA synthesis reaction. These DNA fragments may be separately subjected to step (b), or a mixture thereof may be subjected to step (b). The DNA fragments are preferably mixed and subjected to step (b) from the viewpoint of operation efficiency. The method of the present invention can therefore include a step of mixing the obtained DNA fragments successively after step (a).

After the DNA synthesis reaction, the template DNA may be removed by treatment with a restriction enzyme such as DpnI which digests only methylated DNA, as needed. Alternatively, sufficiently reducing the concentration of the template DNA to be subjected to the DNA synthesis reaction enables the following step to be advanced without the DpnI treatment. After the DNA synthesis reaction, the DNA fragments may be purified by ordinary means such as ethanol precipitation, electrophoresis, column purification, bead purification, or affinity purification as needed.

The thus obtained DNA fragments are DNA fragments which typically form double-stranded circular DNAs consisting of the elongated strands derived from the first primers and the elongated strands derived from the second primers that constitute the mutagenic primer pairs and have 5'-protruding ends, and contain the mutations derived from the mutagenic sites contained in the mutagenic primer pairs.

Alternatively, the following step (a') may be performed as step (a):
(a') subjecting a template DNA and a mutagenic primer pair to DNA synthesis reaction with a DNA polymerase to acquire DNA fragments having 5'-protruding ends, wherein the template DNA is a mixture of a plurality of DNAs having partially different nucleotide sequences, the mutagenic primer pair anneals to the template DNA, primers of the primer pair have regions complementary to each other, and at least one of the primers contains a mutagenic site.

Step (a') is a step of subjecting the template DNA and the mutagenic primer pair to the DNA synthesis reaction with a DNA polymerase to acquire the DNA fragments having 5'-protruding ends. The DNA fragments mutated at specified positions can be obtained by such a step.

The "template DNA" is the mixture of the plurality of DNAs having partially different nucleotide sequences, particularly partially different nucleotide sequence of the DNA of interest. The template DNAs are specifically as described above. The number of the plurality of template DNAs used here is at least 2, preferably 50 or more, more preferably 100 or more, further more preferably 300 or more, still more preferably 500 or more, further still more preferably 1,000 or more, further still more preferably 2 000 or more, further still more preferably 3,000 or more, further still more preferably 5,000 or more, and further still more preferably 10,000 or more, and the number is preferably equal to or less than the number of all the mutants which can be theoretically produced from the nucleotide sequence of the DNA of interest. As the number of the types of template DNAs increases, the mutation library pool obtained becomes more highly diverse.

Alternatively, a mutation library pool prepared by the method of the present invention or a mutation library pool prepared by a well-known method (for example, error-prone PCR) can be also used as the template, and this enables preparing a more highly diverse mutation library pool thereby.

The "mutagenic primer pair" anneals to the template DNA, primers of the primer pair have regions complementary to each other, and at least one of the primers contains a mutagenic site. The mutagenic primer pair is specifically as described above. Only one mutagenic primer pair may be used, or a plurality of mutagenic primer pairs may be used. The number of the plurality of primer pairs used here is at least 2, preferably 10 or more, more preferably 20 or more, further more preferably 30 or more, still more preferably 50 or more, further still more preferably 70 or more, further still more preferably 100 or more, further still more preferably 150 or more, further still more preferably 200 or more, and further still more preferably 300 or more, and the number is preferably equal to or less than the number of types of mutations which can be theoretically introduced into the nucleotide sequence of the DNA of interest. If the plurality of mutagenic primer pairs are used, each mutagenic primer pair is preferably subjected to the DNA synthesis reaction with a DNA polymerase. The DNA synthesis reaction with a DNA polymerase is specifically as described above.

In the method of the present invention, step (b) is a step of allowing a DNA ligase to act on the DNA fragments obtained in step (a). As described above, the DNA fragments obtained in step (a) have 5'-protruding ends, and the 5'-end and the 3'-end of each strand of the DNA fragments are unconnected. Step (b) is a step of ligating the 5'-end of each strand of the DNA fragments and the 3'-end thereof. Such a step enables the efficiency in the subsequent transformation to be greatly improved.

The step of allowing the DNA ligase to act on the DNA fragments can be performed according to a known method. Examples of the DNA ligase which can be used in the method of the present invention include, but not particularly limited to, T4 DNA ligase, *E. coli* DNA ligase, and *Tag* DNA ligase. These DNA ligases are marketed, and can be purchased from TOYOBO Co., Ltd., Takara Bio Inc., and the like. The ligation reaction using the DNA ligase can be performed in an ordinary procedure, for example, according to a protocol attached to the enzyme. The reaction conditions can be appropriately determined depending on the optimal conditions for the enzyme and the amounts of the DNA fragments which are substrates. For example, the ligation reaction can be performed with the temperature adjusted to from 4 to 25°C, preferably 16°C and the reaction time adjusted to from 5 to 60 minutes, preferably from 5 to 30 minutes. Alternatively, the ligation reaction may be performed using a commercially available kit such as Ligation high Ver. 2 (TOYOBO Co., Ltd.) or DNA Ligation Kit Ver. 2.1 (Takara Bio Inc.) according to the protocol attached to the kit.

Examples of a technique for allowing the DNA ligase to act on the DNA fragments efficiently include a technique involving performing step (a) using the mutagenic primer pairs having the 5'-ends phosphorylated beforehand to allow the DNA ligase to act on the obtained DNA fragments. Since phosphate groups are present at the 5'-ends of each strand of the DNA fragments obtained in step (a) at this time, allowing the DNA ligase to act on the DNA fragments efficiently ligates the 5'-ends of each strand of the DNA fragments and the 3'-ends thereof. Examples of the technique include a technique involving performing step (a) with the mutagenic primer pairs having 5'-ends not phosphorylated, allowing polynucleotide kinase to act on the obtained DNA fragments, and subsequently allowing the DNA ligase to act on the obtained DNA fragments. Although phosphate groups are not present at the 5'-ends of each strand of the DNA fragments obtained in step (a) at this time, allowing the polynucleotide kinase to act on the DNA fragments and phosphorylate the 5'-ends of the DNA fragments, and then allowing the DNA ligase to act on the 5'-ends of the DNA fragments efficiently ligates the 5'-ends and the 3'-end thereof. Although these techniques were known as techniques for allowing a DNA ligase to act efficiently, for example, on a DNA fragment ligating into a vector, it is not known that the techniques dramatically improve efficiency in transforming host cells with the DNA fragments obtained using the complementary primer pair(s) as in the present invention, and the fact was completely unexpected.

Furthermore, examples of the techniques for allowing the DNA ligase to act on the DNA fragments efficiently also include a technique involving adding a DNA ligase to the DNA fragments obtained in step (a) and allowing 5' exonuclease and DNA polymerase to act on the DNA fragments in combination. Even if step (a) is performed with the mutagenic primer pairs having the 5'-ends not phosphorylated, the 5'-end of each strand of the obtained DNA fragments is efficiently ligated with the 3'-ends thereof. The reagent NEBuilder (Registered Trademark) HiFi DNA Assembly Master Mix (New England Biolabs Japan Inc.), containing the three above-mentioned three enzymes, is marketed. Although it is known that the reagent enables various DNA fragments to be assembled, it is not known that the reagent dramatically improves efficiency in transforming host cells with the DNA fragments obtained using the complementary primer pair(s) as in the present invention, and the fact was completely unexpected.

If each of the DNA fragments obtained in step (a) are separately subjected to step (b), the obtained DNAs may be subjected to step (c) separately or mixed to subject to step (C), and the DNA fragments may be preferably mixed to subject to step (c) from the viewpoint of operation efficiency. The method of the present invention can therefore include a step of mixing the obtained DNAs successively after step (b).

The DNAs obtained in step (b) may be purified by ordinary means such as ethanol precipitation, electrophoresis, column purification, bead purification, or affinity purification as needed.

In the method of the present invention, step (c) is a step of transforming host cells with the DNAs obtained in step (b). The step can be performed to obtain transformants containing the mutated DNAs of interest.

Examples of the host cells include microorganisms such as bacteria and filamentous fungi. Examples of the bacteria include *Escherichia coli* and bacteria belonging to *Staphylococcus, Enterococcus, Listeria,* and *Bacillus.* Among these, *Escherichia coli* and *Bacillus* bacteria are preferable, and *Escherichia coli* is more preferable. Examples of the filamentous fungi include fungi belonging to *Trichoderma, Aspergillus,* and *Rhizopus.*

As the transformation method, methods such as electroporation, transformation, transfection, conjugation, a protoplast method, a particle gun method, and an *Agrobacterium* method commonly used in the art are usable. Suitable transformants (suitably transformed cells) can be selected using, for example, marker gene expression or auxotrophy as an indicator.

If each DNA fragment obtained in step (a) is separately subjected to steps (b) and (c), the selected transformants may be mixed. The method of the present invention can therefore include a step of mixing the obtained transformants successively after step (c).

The selected transformants are bacterial cells or fungal cells containing the DNAs of interest containing the different nucleotide sequences due to the different mutations. The mixture of the selected transformants therefore constitutes a mutation library pool. DNAs extracted from a mixture of the selected transformants or a mixture of or DNAs extracted from the selected transformants also constitute a mutation library pool. The DNAs can be extracted or isolated from the transformants or a mixture of the transformants by an ordinary manner in the art to acquire the DNAs. For example, a commercially available DNA extraction kit can be used for the extraction or the isolation.

A mutation library pool having further enhanced diversity can be prepared by the method for preparing a mutation library pool of the present invention or a known method for preparing a mutation library pool (for example, error-prone PCR) using the mutation library pool prepared by the method of the present invention as templates.

In one preferable embodiment of the method of the present invention, the template DNA contains a DNA encoding a protein to be mutated. The mutagenic site contained in at least one primer of mutagenic primer pairs preferably consists of a nucleotide sequence that induces substitution mutation of an amino acid residue at a specified position of the protein. The nucleotide sequence such as NNK, NNS, NNB, NNN, NDT, and DBK are more preferably used. The nucleotide sequence of the mutagenic site which is NNK, NNS, NNB, NNN, NDT, or DBK can introduce codons specifying a plurality of amino acid residues. The nucleotide sequence of the mutagenic site which is especially NNK, NNS, or NNB can comprehensively introduce codons specifying 20 amino acid residues and termination codons, and enables the termination codons to be less likely to be generated. The DNA fragments obtained by subjecting the template DNA and the mutagenic primer pairs having the nucleotide sequences of the above-mentioned mutagenic site which is NNK, NNS, or NNB to the DNA synthesis reaction with a DNA polymerase are a mixture of DNA fragments in which the codon encoding the amino acid residue at the specified position of the protein is a codon encoding the original amino acid residue, a codon encoding any amino acid residue other than the original amino acid residue, or a termination codon, consequently providing saturation mutagenesis of the amino acid residue at the specified position of the protein. An increase in the number of the mutagenic primer pairs having the nucleotide sequences of the above-mentioned mutagenic site which is NNK, NNS, or NNB therefore leads to increase in saturation mutagenesis of the amino acid residues in the amino acid sequence of the protein. The number of the above-mentioned mutagenic primer pairs used here is preferably at least 2, more preferably 10 or more, further more preferably 20 or more, still more preferably 30 or more, further still more preferably 50 or more, further still more preferably 70 or more, further still more preferably 100 or more, further still more preferably 150 or more, further still more preferably 200 or more, and further still more preferably 300 or more, and the number is preferably equal to or less than the number of the amino acid residues of the protein. The mutagenic primer pairs in which the nucleotide sequence of the above-mentioned mutagenic site is NNK, NNS, or NNB for each of the amino acid residues of the protein (namely the mutagenic primer pairs which are equal to the amino acid residues of the protein in number) can be used to prepare a single mutation library pool of the protein. The single mutation library pool can be used as the template DNA to produce a double mutation library pool of the protein.

The mutation library pool prepared by the method of the present invention has a DNA sequence diversity of preferably 10⁴ or more, more preferably 10⁵ or more, further more preferably 10⁶ or more, and still preferably 10⁷ or more. For example, the nucleotide sequences of each member constituting the mutation library pool can be sequenced to confirm the DNA sequence diversity based on the obtained sequence data. The DNA sequence diversity can be expressed as a theoretical DNA sequence diversity roughly calculated based on the number of the template DNAs, the number of the mutagenic primer pairs, and the number of types of mutations to be introduced with the mutagenic primer pairs. In one example, if saturation mutations is introduced into all the single amino acid residues in DNA encoding a protein having a full length of 500 amino acids using mutagenic primer pairs in which the nucleotide sequence of the mutagenic sites is NNK, the sequences represented by NNK include 4 × 4 × 2 = 32 sequences, and the theoretical DNA sequence diversity of the obtained single mutation library pool can therefore be roughly calculated to be 500 × 32 = 1.6 × 10⁴. In another example, if saturation mutations are introduced into two amino acid residues among all the combinations in DNA encoding a full length of 500 amino acids using mutagenic primer pairs in which the nucleotide sequence of the mutagenic sites is NNK, the theoretical DNA sequence diversity of the obtained double mutation library pool can be roughly calculated to be ₅₀₀C₂ × 32² = around 1.3 × 10⁸.

As shown in the following Examples, while a single mutation library pool of a protein having a full length of 483 amino acids and obtained by the method of the present invention had a theoretical DNA sequence diversity of around 1.5 × 10⁴ and an amino acid sequence diversity of around 9.2 × 10³, the library size calculated from the transformation efficiency was 2.7 × 10⁸, and the single mutation library pool had so high diversity as to enables diversity of all the single mutations to be comprehended. This diversity is three times or more as high as the theoretical diversity of a single mutation library pool which can be prepared by error-prone PCR utilized for preparing a mutation library pool until now. The average number of mutations introduced into the single mutation library pool was 0.91, and was a good value close to 1, namely the theoretical value. Furthermore, the single mutation library pool covered as high as 80% of the theoretical DNA sequence diversity. Meanwhile, while a double mutation library pool of the protein had a theoretical DNA sequence diversity of around 1.1 × 10⁸, and an amino acid sequence diversity of around 4.2 × 10⁷, the library size calculated from the transformation efficiency was 9.5 × 10⁷, and the double mutation library pool had so high diversity as to enables most diversity of all the double mutations to be comprehended. The average number of mutations introduced into the double mutation library pool was 1.73, and was a good value close to 2, namely the theoretical value. Furthermore, the double mutation library pool covered as high as 80% of the theoretical DNA sequence diversity. Thus, the mutation library pool obtained by the method of the present invention is highly diverse, and includes mutations of interest widely.

The mutation library pool can be used to screen for a DNA sequence for analyzing or improving function of the DNA sequences. For example, DNA having a desired property can be selected from the mutation library pool prepared by the method of the present invention using the desired property as an index. The "property" of the DNA as used here can be expressed, for example, in the activity of the polypeptide (for example, enzyme) encoded by the DNA, the stability of a polypeptide encoded by the DNA, the expression level of the polypeptide encoded by the DNA, the activity of a promoter encoded by the DNA, the copy number of plasmids containing the DNA, and the expression level of the polypeptide encoded by DNA in a region different from the DNA in the plasmid containing the DNA. The mutation library pool therefore enables DNA to be selected, for example, wherein the activity of the encoded polypeptide is high or low, the stability of the encoded polypeptide is high or low, the expression level of the encoded polypeptide is high or low, the activity of the encoded promoter is high or low, the copy number of plasmids containing the DNA is large or small, or the expression level of the polypeptide encoded by DNA in a region different from the region of the DNA in the plasmid containing the DNA is high or low. The DNA having the desired property can be appropriately selected from the mutation library pool depending on the desired property according to an ordinary method.

As illustrative embodiments of the present invention, the following substances, production methods, uses, methods, and the like will be further disclosed herein. The present invention is not, however, limited to these embodiments.

[1] A method for preparing a mutation library pool, comprising the following steps (a) to (c):
   (a) subjecting a template DNA and each of a plurality of mutagenic primer pairs to DNA synthesis reaction with a DNA polymerase to acquire DNA fragments having 5'-protruding ends, wherein each of the mutagenic primer pairs anneals to the template DNA, primers of each primer pair have regions complementary to each other, and at least one of the primers comprises a mutagenic site;
   (b) allowing a DNA ligase to act on the DNA fragments obtained in step (a); and
   (c) transforming host cells with the DNAs obtained in step (b).
[2] The method according to [1], wherein preferably at least 2, more preferably 10 or more, further more preferably 20 or more, still more preferably 30 or more, further still more preferably 50 or more, further still more preferably 70 or more, further still more preferably 100 or more, further still more preferably 150 or more, further still more preferably 200 or more, and further still more preferably 300 or more of the mutagenic primer pairs are used.
[3] The method according to [1] or [2], wherein the template DNA is a mixture of preferably a plurality of DNAs having partially different nucleotide sequences, namely a mixture of at least 2, more preferably 50 or more, further more preferably 100 or more, still more preferably 300 or more, further still more preferably 500 or more, further still more preferably 1 000 or more, further still more preferably 2 000 or more, further still more preferably 3 000 or more, further still more preferably 5 000 or more, and further still more preferably 10 000 or more of DNAs.
[4] The method according to any one of [1] to [3], further comprising the steps of:
   mixing the DNA fragments obtained in step (a);
   mixing the DNAs obtained in step (b); or
   mixing the transformants obtained in step (c).
[5] The method according to any one of [1] to [4], wherein the template DNA preferably comprises DNA encoding a protein to be mutated.
[6] The method according to any one of [1] to [5], wherein a nucleotide sequence at the mutagenic site is preferably NNK, NNS, NNB, NNN, NDT, or DBK and more preferably NNK, NNS, or NNB.
[7] The method according to [5] or [6], wherein the mutagenic primer pairs having the number equal to the number of the amino acid residues of the protein is preferably used.
[8] The method according to any one of [1] to [7], wherein the mutation library pool has a DNA sequence diversity of preferably 10⁴ or more, more preferably 10⁵ or more, further more preferably 10⁶ or more, and still more preferably 10⁷ or more.
[9] A method for preparing a mutation library pool, comprising the following steps (a) to (c):
   (a) subjecting a template DNA and each of a plurality of mutagenic primer pairs to DNA synthesis reaction with a DNA polymerase to acquire DNA fragments having 5'-protruding ends, wherein the template DNA is the mutation library pool prepared by the method according to any one of [1] to [8], each of the mutagenic primer pairs anneals to the template DNA, primers of each primer pair have regions complementary to each other, and at least one of the primers comprises a mutagenic site;
   (b) allowing a DNA ligase to act on the DNA fragments obtained in step (a); and
   (c) transforming host cells with the DNAs obtained in step (b).
[10] A method for preparing a mutation library pool, comprising the following steps (a') to (c):
   (a') subjecting a template DNA and a mutagenic primer pair to DNA synthesis reaction with a DNA polymerase to acquire DNA fragments having 5'-protruding ends, wherein the template DNA is a mixture of a plurality of DNAs having partially different nucleotide sequences, the mutagenic primer pair anneals to the template DNA, primers of each primer pair have regions complementary to each other, and at least one of the primers comprises a mutagenic site;
   (b) allowing a DNA ligase to act on the DNA fragments obtained in step (a'); and
   (c) transforming host cells with the DNAs obtained in step (b).
[11] The method according to [10], wherein the template DNA is a mixture of at least 2, more preferably 50 or more, further more preferably 100 or more, still more preferably 300 or more, further still more preferably 500 or more, further still more preferably 1,000 or more, further still more preferably 2,000 or more, further still more preferably 3,000 or more, further still more preferably 5,000 or more, and further still more preferably 10,000 or more of DNAs having partially different nucleotide sequences.
[12] The method according to [10] or [11], wherein the template DNA is a mutation library pool prepared by the method according to any one of [1] to [9].
[13] The method according to any one of [10] to [12], wherein preferably at least 2, more preferably 10 or more, further more preferably 20 or more, still more preferably 30 or more, further still more preferably 50 or more, further still more preferably 70 or more, further still more preferably 100 or more, further still more preferably 150 or more, further still more preferably 200 or more, and further still more preferably 300 or more of the mutagenic primer pairs are used, and each of the mutagenic primer pairs is subjected to the DNA synthesis reaction with a DNA polymerase.
[14] The method according to [13], further comprising the steps of:
   mixing the DNA fragments obtained in step (a');
   mixing the DNAs obtained in step (b); or
   mixing the transformants obtained in step (c).
[15] The method according to any one of [10] to [14], wherein the template DNA preferably comprises the DNA encoding the protein to be mutated.
[16] The method according to any one of [10] to [15], wherein a nucleotide sequence at the mutagenic site is preferably NNK, NNS, NNB, NNN, NDT, or DBK and more preferably NNK, NNS, or NNB.
[17] The method according to [15] or [16], wherein the mutagenic primer pairs having the number equal to the number of the amino acid residues of the protein is preferably used.
[18] The method according to any one of [10] to [17], wherein the prepared mutation library pool has a DNA sequence diversity of preferably 10⁴ or more, more preferably 10⁵ or more, further more preferably 10⁶ or more, and still more preferably 10⁷ or more.
[19] A mutation library pool prepared by the method according to any one of [1] to [18].
[20] The mutation library pool according to [19], wherein the DNA sequence diversity is preferably 10⁴ or more, more preferably 10⁵ or more, further more preferably 10⁶ or more, and still more preferably 10⁷ or more.
[21] A method for screening for DNA having a desired property, comprising a step of selecting DNA having the desired property from the mutation library pool prepared by the method according to any one of [1] to [18] using the desired property as an indicator.
[22] The method according to [21], wherein the property of the DNA is expressed in activity of a polypeptide encoded by the DNA, stability of the polypeptide encoded by the DNA, an expression level of the polypeptide encoded by the DNA, activity of a promoter encoded by the DNA, a copy number of plasmids containing the DNA, the expression level of the polypeptide encoded by the DNA in a region different from a region of the DNA in the plasmid containing the DNA.

### Examples

Although the present invention will be described based on the Examples in further detail hereinafter, the present invention is not limited to these Examples.

### Example 1 Design of primer pairs for saturation mutagenesis

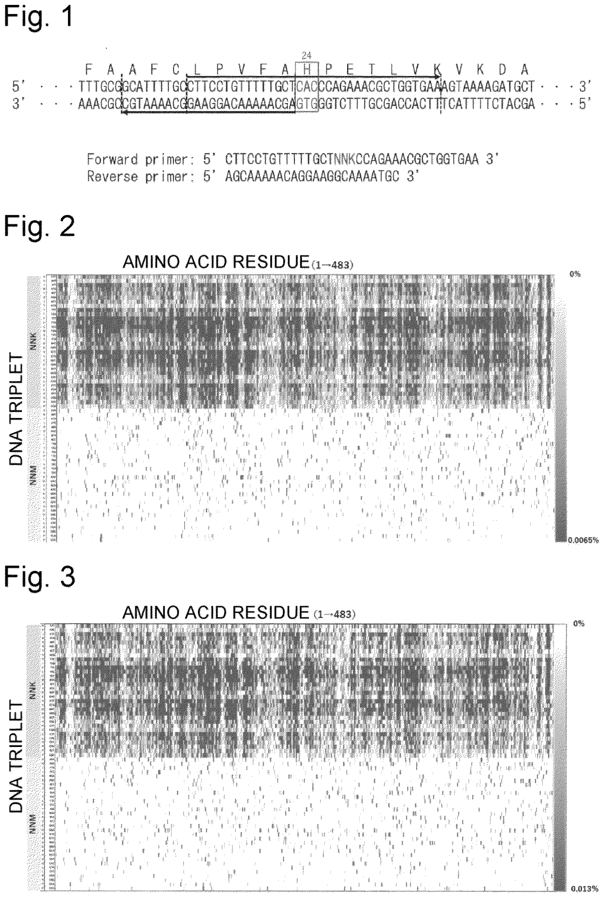

Sequences in which the codon of an amino acid to be mutated was changed into NNK in a total of 35 bases consisting of the codon added with 15 bases upstream thereof, and 17 bases downstream thereof was used as forward primers. Sequences complementary to 24 bases upstream of the codon of the amino acid to be mutated was used as reverse primers. The primer pairs are therefore complementary to each other in a region of 15 bases upstream of the codon of the amino acid to be mutated. As an example, Figure 1 shows an outline of the design of primers for saturation mutagenesis of the histidine at residue position 24 of β-lactamase encoded as an ampicillin resistance gene in the plasmid pHY300PLK (Takara Bio Inc.). SEQ ID NO: 1 shows the amino acid sequence of β-lactamase, SEQ ID NO: 2 shows the nucleotide sequence encoding β-lactamase, and SEQ ID NOS: 3 and 4 show the nucleotide sequences of forward primers for saturation mutagenesis of the histidine at residue position 24 of β-lactamase (CTTCCTGTTTTTGCTNNKCCAGAAACGCTGGTGAA) and the nucleotide sequence of a reverse primer therefor (AGCAAAAACAGGAAGGCAAAATGC), respectively.

### Example 2 Site-specific mutation by PCR using complementary primer pairs

A total of six sequences of primers for saturation mutagenesis of residues at positions 24, 25, and 26 of β-lactamase encoded as an ampicillin resistance gene in the plasmid pHY300PLK were designed in the same way as in Example 1, and the oligo DNAs were synthesized. The plasmid pHY300PLK which was template DNA and each primer pair were subjected to PCR reaction according to the recommended protocol of KOD One (TOYOBO Co., Ltd.). The amplification of the fragments of interest was confirmed by electrophoresis. All the PCR reaction solutions were then mixed, followed by the digestion of the template with DpnI. The PCR fragments were purified with a High Pure PCR Product Purification Kit (Roche Diagnostics K.K.). E. cloni(R) 10G SUPREME Electrocompetent Cells (SOLOs) (LGC), which were *Escherichia coli* competent cells for highly efficiently constructing libraries, were transformed with 100 ng of this DNA according to the recommended protocol for the kit. The plasmid pHY300PLK, used as the template, contains a tetracycline resistance gene besides the ampicillin resistance gene. Even if pHY300PLK loses ampicillin resistance, the plasmid can therefore be proliferated in tetracycline-containing medium. The transformed bacterial solution was appropriately diluted, and the dilution was applied to tetracycline-containing LB agar medium. The resultants were incubated at 37°C overnight, and the formed colonies were then counted to calculate the transformation efficiency (CFU). The transformation efficiency was 8.2 × 10⁴ CFU. This is insufficient transformation efficiency for constructing a mutation library pool having a diversity of 10⁵ or more.

### Example 3 Improvement in transformation efficiency 1

The 5'-ends of the primers in Example 2 were subjected to phosphorylation modification beforehand, and PCR fragments were prepared in the same way. The PCR fragments were subjected to ligation reaction according to the protocol recommended for Ligation high Ver. 2 (TOYOBO Co., Ltd.), followed by purification using ethanol precipitation. Transformation was carried out with 100 ng of this DNA in the same way as in Example 2, and the transformation efficiency was determined. The transformation efficiency was 3.8 × 10⁶ CFU. When the sequences of the plasmids were confirmed in multiple colonies, mutation was introduced at any of residue positions 24, 25, and 26 of β-lactamase.

The above-mentioned results showed that, in the site-directed mutagenesis method by PCR using the complementary primer pairs, the combination of the ligation reactions dramatically improved the transformation efficiency to enable a large-scale mutation library pool to be prepared.

### Example 4 Improvement in transformation efficiency 2

The PCR fragments purified in Example 2 was mixed with NEBuilder HiFi DNA Assembly Master Mix (New England Biolabs Japan Inc.), having 5'-exonuclease activity, DNA polymerase activity, and DNA ligase activity, in equal amounts. The mixture was incubated at 50°C for 1 hour and then purified by ethanol precipitation. Transformation was carried out with 100 ng of this DNA in the same way as in Example 2, and the transformation efficiency was determined. The transformation efficiency was 9.3 × 10⁷ CFU. When the sequences of the plasmids were confirmed in multiple colonies, mutation was introduced at any of residue positions 24, 25, and 26 of β-lactamase.

The above-mentioned results showed that the combination of 5'-exonuclease activity, DNA polymerase activity, and DNA ligase activity also dramatically improved the transformation efficiency in the PCR products obtained using the complementary primer pairs not subjected to phosphorylation modification to enable a large-scale mutation library pool to be prepared.

### Example 5 Construction of expression plasmid YR288dRT

SEQ ID NO: 5 shows the amino acid sequence of YR288dRT, which is a mutant consisting of 483 amino acids wherein the R178 and T180 positions of α-amylase derived from *Bacillus* sp. strain YR288 (WP_100346362.1) were deleted. SEQ ID NO: 6 shows the nucleotide sequence of DNA encoding YR288dRT. The primers stop_pET22b_fw (TGAGATCCGGCTGCTAACAAAGCCC: SEQ ID NO: 7) and pelB_rv (GGCCATCGCCGGCTGGGC: SEQ ID NO: 8) were subjected to PCR using pET22b(+) (Novagen) as a template to amplify a vector fragment containing the PelB signal and a stop codon. The primers pelB_YR288_fw (CAGCCGGCGATGGCCGCAGCTCAAAACGGTACGATGATGCAG: SEQ ID NO: 9) and pET22b_YR288_rv (AGCAGCCGGATCTCACTGTTGAACGTACACGGAGACTGAGC: SEQ ID NO: 10) were subjected to PCR using the DNA encoding YR288dRT as a template to amplify a fragment encoding YR288dRT. These fragments were subjected to In-Fusion reaction with an In-Fusion (Registered Trademark) HD Cloning Kit (Takara Bio Inc.), and the obtained In -Fusion reaction solution was transformed into ECOSTM Competent *E. coli* DH5 α (Nippon Gene Co., Ltd.) to construct the plasmid pET22b(+)-YR288dRT.

### Example 6 Preparation of single mutation library pool of YR288dRT

As two sequences of a forward primer and a reverse primer are used, a total of 966 sequences of primers for saturation mutagenesis of 483 amino acids corresponding to the full length of YR288dRT using pET22b(+)-YR288dRT as a template were designed in the same way as in Example 1 to synthesize oligo DNAs. As an example, SEQ ID NOS: 11 and 12 show the nucleotide sequence of a forward primer (GGTACGATGATGCAGNNKTTTGAATGGTATGTACC) and the nucleotide sequence of a reverse primer (CTGCATCATCGTACCGTTTTGAGC), respectively, for saturation mutagenesis of the tyrosine residue at position 10 of YR288dRT. The plasmid pET22b(+)-YR288dRT which was a template DNA and each primer pair were subjected to a total of 483 PCR reactions according to the recommended protocol of KOD One (TOYOBO Co., Ltd.). The amplification of the fragments of interest was confirmed by electrophoresis. All the PCR reaction solutions were then mixed, and the template was digested with DpnI. The PCR fragments were then purified with a High Pure PCR Product Purification Kit (Roche Diagnostics K.K.). The purified fragments were mixed with NEBuilder HiFi DNA Assembly Master Mix (New England Biolabs Japan Inc.) in equal amounts. The mixture was incubated at 50°C for 1 hour, followed by purification using ethanol precipitation. Transformation was carried out with 1 µg of this DNA in the same way as in Example 2. Then, 1 µL of the transformed bacterial solution was appropriately diluted, and the dilution was applied to ampicillincontaining LB agar medium, followed by incubation at 37°C overnight. The formed colonies were counted to calculate the transformation efficiency (CFU). The whole amount (around 1 mL) of the remaining bacterial solution was inoculated into 20 mL of 50 ppm Carbenicillin-containing LB medium, and cultured at 37°C overnight. Plasmids were then extracted and used as a single mutation library pool of YR288dRT. The theoretical DNA sequence diversity of the single mutation library pool of YR288dRT was 1.5 × 10⁴, the theoretical amino acid sequence diversity was 9.2 × 10³, and the library size calculated from the transformation efficiency was 2.7 × 10⁸. It was believed from these results that the generated mutation library pool was able to comprehend all the diverse single mutations fully.

### Example 7 Preparation of double mutation library pool of YR288dRT

The same primer set as the primer set used in Example 6 was subjected to a total of 483 PCR reactions using the single mutation library pool of YR288dRT prepared in Example 6 as a template DNA. The amplification of the fragments of interest was confirmed by electrophoresis. All the PCR reaction solutions were then mixed, and the template was digested with DpnI. The PCR fragments were then purified with a High Pure PCR Product Purification Kit (Roche Diagnostics K.K.). The purified fragments were mixed with NEBuilder HiFi DNA Assembly Master Mix (New England Biolabs Japan Inc.) in equal amounts. The mixture was incubated at 50°C for 1 hour, followed by purification using ethanol precipitation. Transformation was carried out with 1 µg of this DNA in the same way as in Example 2. Then, 1 µL of the transformed bacterial solution was appropriately diluted, and the dilution was applied to ampicillincontaining LB agar medium, followed by incubation at 37°C overnight. The formed colonies were counted to calculate the transformation efficiency (CFU). The whole amount (around 1 mL) of the remaining bacterial solution was inoculated into 20 mL of 50 ppm Carbenicillin-containing LB medium, and cultured at 37°C overnight. Plasmids were then extracted and used as a double mutation library pool of YR288dRT. The theoretical DNA sequence diversity of the double mutation library pool of YR288dRT was 1.2 × 10⁸, the theoretical amino acid sequence diversity was 4.2 × 10⁷, and the library size calculated from the transformation efficiency was 9.5 × 10⁷. It was believed from these results that the generated mutation library pool was able to comprehend most of the diverse double mutations fully.

### Example 8 Confirmation of quality of mutation library pool of YR288dRT

The primers pET22b_ NGS_fw (GCGAAATTAATACGACTCACTATAG: SEQ ID NO: 13) and pET22b_NGS_rv (GCCAATCCGGATATAGTTCC: SEQ ID NO: 14) were subjected to PCRs using pET22b(+)-YR288dRT, the single mutation library pool of YR288dRT prepared in Example 6, and the double mutation library pool of YR288dRT prepared in Example 7 as templates to amplify fragments containing the full lengths of the coding regions of YR288dRT (and mutants thereof). Each PCR products was subjected to agarose gel electrophoresis, and only band portions of interest in the gel were then cut out, followed by purification using High Pure PCR Product Purification Kit (Roche Diagnostics K.K.). The purified DNAs were fragmented according to the recommended protocol of NEBNext (Registered Trademark) dsDNA Fragmentase (New England Biolabs Japan Inc.), and libraries for a nextgeneration sequencer were prepared according to the recommended protocol of a NEBNext Ultra (Treadmark) II DNA Library Prep Kit for Illumina (Registered Trademark) (New England Biolabs Japan Inc.). The concentrations were measured with a GenNext NGS Library Quantification Kit (TOYOBO Co., Ltd.), followed by sequencing with a MiSeq system (Illumina K.K.) using a MiSeq Reagent Micro Kit v2 (300-cycles) (Illumina K.K.). Trimming and resequencing were carried out with a CLC Genomics Workbench (QIAGEN) to output read information.

In each codon, the proportion of the number of reads into which mutations were introduced to reads containing the codon was calculated as a Mutation Rate. The total of the Mutation Rates in the full length is the average number of mutations introduced in each mutation library pool. The total of the Mutation Rates was calculated in each mutation library pool, and a value calculated by subtracting the total of the Mutation Rate of pET22b(+)-YR288dRT (Mutation Rate due to the error of MiSeq) as the blank therefrom was defined as the average number of mutations introduced. The average number of mutations introduced into the single mutation library pool was 0.91, and the average number of mutations introduced into the double mutation library pool was 1.73.

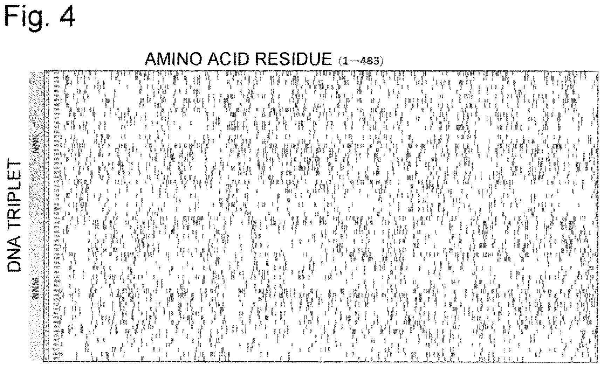

The appearance frequencies of the 64 DNA triplets were calculated in each codon to make heat maps. The theoretical values in the case where it was assumed that mutations were uniformly introduced (0.0065% in the single mutation library pool and 0.013% in the double mutation library pool) were defined as the upper limit values of the heat maps, and the lower limit values were defined as 0. Both the single mutation library pool (Figure 2) and the double mutation library pool (Figure 3) included 32 DNA triplets contained in the mixed base NNK at high coverages. In the single mutation library pool, the number of mutants at appearance frequencies which were equal to or greater than 1/10 as high as the theoretical value 0.0065% was 13401, which exhibited a coverage of 80% of the theoretical DNA sequence diversity. Meanwhile, mutations which were able to be introduced by substituting only 1 base in each codon were mapped as mutants which were able to be accessed by error-prone PCR in Figure 4. Since the number of the mutants which were able to be introduced by substituting only 1 base was 4347, the single mutation library pool has three times or more the single mutation diversity of the mutation library pool which can be prepared by error-prone PCR.

The above-mentioned results showed that the prepared single mutation library pool and double mutation library pool had high diversities, high mutagenesis rates, and high coverages.

## Claims

1. A method for preparing a mutation library pool, comprising the following steps (a) to (c):
(a) subjecting a template DNA and each of a plurality of mutagenic primer pairs to DNA synthesis reaction with a DNA polymerase to acquire DNA fragments having 5'-protruding ends, wherein each of the mutagenic primer pairs anneals to the template DNA, primers of each primer pair have regions complementary to each other, and at least one of the primers comprises a mutagenic site;
(b) allowing a DNA ligase to act on the DNA fragments obtained in step (a); and
(c) transforming host cells with the DNAs obtained in step (b).

2. The method according to claim 1, wherein 10 or more mutagenic primer pairs are used.

3. The method according to claim 1 or 2,
wherein the template DNA is a mixture of a plurality of DNAs having partially different nucleotide sequences.

4. The method according to any one of claims 1 to 3, further comprising the steps of: mixing the DNA fragments obtained in step (a); mixing the DNAs obtained in step (b); or mixing the transformants obtained in step (c) .

5. A method for preparing a mutation library pool, comprising the following steps (a) to (c):
(a) subjecting a template DNA and each of a plurality of mutagenic primer pairs to DNA synthesis reaction with a DNA polymerase to acquire DNA fragments having 5'-protruding ends, wherein the template DNA is the mutation library pool prepared by the method according to any one of claims 1 to 4, each of the mutagenic primer pairs anneals to the template DNA, primers of each primer pair have regions complementary to each other, and at least one of the primers comprises a mutagenic site;
(b) allowing a DNA ligase to act on the DNA fragments obtained in step (a); and
(c) transforming host cells with the DNAs obtained in step (b).

6. A method for preparing a mutation library pool, comprising the following steps (a') to (c):
(a') subjecting a template DNA and a mutagenic primer pair to DNA synthesis reaction with a DNA polymerase to acquire DNA fragments having 5'-protruding ends, wherein the template DNA is a mixture of a plurality of DNAs having partially different nucleotide sequences, the mutagenic primer pair anneals to the template DNA, primers of the primer pair have regions complementary to each other, and at least one of the primers comprises a mutagenic site;
(b) allowing a DNA ligase to act on the DNA fragments obtained in step (a'); and
(c) transforming host cells with the DNAs obtained in step (b).

7. The method according to claim 6, wherein the template DNA is a mixture of 50 or more DNAs having partially different nucleotide sequences.

8. The method according to claim 6 or 7, wherein the template DNA is the mutation library pool prepared by the method according to any one of claims 1 to 5.

9. A mutation library pool prepared by the method according to any one of claims 1 to 8.

10. A method for screening for DNA having a desired property, comprising a step of selecting DNA having the desired property from the mutation library pool prepared by the method according to any one of claims 1 to 9 using the desired property as an indicator.
